# FASCICULE DE BREVET EUROPEEN

(11) **EP 2 226 089 B1**
(45) Date de publication et mention de la délivrance du brevet: **31.07.2013**
(21) Numéro de dépôt: 09002255.9
(22) Date de dépôt: 18.02.2009
(51) Int. Cl.: A61M 5/32

(54) **Seringue de sécurité**
Sicherheitsspritze
Safety syringe

(43) Date de publication de la demande: 08.09.2010
(73) Titulaire: Loretti, Maurice, 1219 Chatelaine (CH); Djokic, Savka, 1007 Lausanne (CH)
(72) Inventeur: Loretti, Maurice, 1219 Chatelaine (CH); Djokic, Savka, 1007 Lausanne (CH)
(74) Mandataire: KIRKER & Cie S.A.

(56) Documents cités:
- EP-A- 1 797 921
- WO-A-2008/004114
- FR-A- 2 791 264
- US-A1- 2004 236 283
- US-A1- 2007 250 015

## Description

La présente invention a pour objet une seringue pour injection hypodermique, intramusculaire ou intraveineuse qui soit d'une utilisation simple, d'un coût ne dépassant pas celui des seringues usuelles non protégées et qui apporte une facilité d'utilisation et une sécurité accrue au personnel soignant pour éviter à celui-ci des accidents encore trop fréquents et interdire également une réutilisation de la seringue.

Le but de la présente invention est de réaliser une seringue de sécurité évitant les blessures du personnel soignant et interdisant une réutilisation, qui puisse être fabriquée à faible coût. Un autre but de la présente invention est de réaliser une seringue dont le piston soit exempt de joint en élastomère susceptible de relâcher des particules et rendant impraticable l'injection du médicament.

On connaît du document W02008/004114 une seringue de sécurité comportant un cylindre et un piston muni d'un joint en élastomère, ce cylindre comportant une partie proximale, une partie distale destinée à recevoir une aiguille et une partie intermédiaire dont le diamètre externe est plus petit que le diamètre interne de la partie proximale de grand diamètre du cylindre. Dans cette seringue la paroi frontale du cylindre reliant la partie proximale à sa partie intermédiaire comporte une zone d'affaiblissement ou de rupture et la partie distale du piston comporte une première formation d'accouplement axial coopérant avec une seconde formation d'accouplement axial que présente la partie intermédiaire de ce cylindre. Ces première et seconde formations d'accouplement axial permettent un accouplement irréversible entre le piston et la partie intermédiaire du cylindre.

Cette seringue représente indéniablement un progrès par rapport aux seringues de sécurité connues avant 2006 mais présente néanmoins l'inconvénient de comporter un joint élastomère et de requérir du personnel soignant une attention particulière pour pouvoir accoupler le piston à la partie intermédiaire du cylindre qui ne peut se faire que lorsque ces parties sont angulairement orientées l'une par rapport à l'autre. L'utilisateur doit donc en fin d'injection orienter le piston angulairement par rapport au cylindre pour pouvoir accoupler ces deux pièces, ce qui requiert dextérité et perte de temps. Ceci peut aussi dans certains cas conduire à ne pas accoupler ces deux parties et donc à ne pas sécuriser la seringue.

Cette seringue décrite dans le document W02008/004114 présente encore l'inconvénient que si l'utilisateur ne fait pas très attention il peut, par inadvertance ou volontairement, après l'injection extraire le piston et son aiguille hors du cylindre ce qui peut présenter un danger de blessure pour l'usager. En effet l'éventuelle butée de retenue n'empêche pas l'extraction du piston hors du cylindre mais permet seulement de rendre attentif l'usager que le piston est en fin de course arrière.

Le document EP 1 797 921 décrit également une seringue de sécurité du même type que celle décrite dans le document précédent où la butée de retenue ne permet pas non plus d'exclure la séparation du cylindre et du piston après l'injection.

La seringue selon la présente invention a pour but de remédier aux inconvénients précités pour faciliter encore l'utilisation de la seringue et notamment se passer du joint élastomère. Enfin, un autre but encore de la seringue selon l'invention est d'éviter qu'après l'injection le piston ne puisse être retiré hors du cylindre pour éviter toute blessure du personnel soignant.

La présente invention a pour objet une seringue hypodermique de sécurité tendant à obvier aux inconvénients précités et qui comporte un corps de seringue présentant un cylindre (1) et un piston présentant un joint (15) coulissant à l'intérieur de ce cylindre de façon étanche, dans laquelle le cylindre comporte une partie distale (5) destinée à recevoir une aiguille et une partie intermédiaire (4) dont le diamètre externe est plus petit que le diamètre interne du cylindre (1), la paroi frontale (1a) du cylindre le reliant à la partie intermédiaire (4) comportant une zone de rupture (6), et dans laquelle la partie distale du piston comporte une première formation d'accouplement axial coopérant avec une seconde formation d'accouplement axial que présente la partie intermédiaire (4) du cylindre formant ensemble un accouplement axial irréversible entre le piston et la partie intermédiaire (4) du cylindre (1), cette partie distale du piston comportant en outre une formation d'entraînement en rotation (16, 17) coopérant avec une formation d'entraînement correspondante (4a) que comporte la partie intermédiaire (4) du cylindre (1), le corps de seringue et le piston étant chacun venus d'une seule pièce de fabrication; caractérisée par le fait que l'extrémité arrière du cylindre (1) comporte une portion conique (3) allant en s'évasant vers l'arrière et reliant ledit cylindre à une plaque (2); que cette portion conique (3) comporte des fentes longitudinales (8) délimitant au moins une portion de paroi (9) de cette portion conique (3) susceptible de se déplacer élastiquement radialement; et par le fait que la face interne de ces portions de paroi (9) comporte d'une part une rainure de guidage longitudinale (10) coopérant avec une arête (19) de la tige (13) du piston et d'autre part au moins une nervure (11) longitudinale présentant une rampe progressant de l'arrière de la portion conique (3) en direction du cylindre et se terminant par une butée (11a) située sur un diamètre plus petit que le diamètre intérieur du cylindre (1) et donc du joint (15) du piston de manière à permettre lors du montage de la seringue l'introduction du piston dans le cylindre par déformation élastique de la portion de paroi (9) mais à interdire tout retrait du piston hors du cylindre.

D'autres caractéristiques importantes de la présente seringue sont énoncées dans les revendications dépendantes.

Le dessin annexé illustre schématiquement et à titre d'exemple une forme d'exécution particulière de la seringue de sécurité selon l'invention.

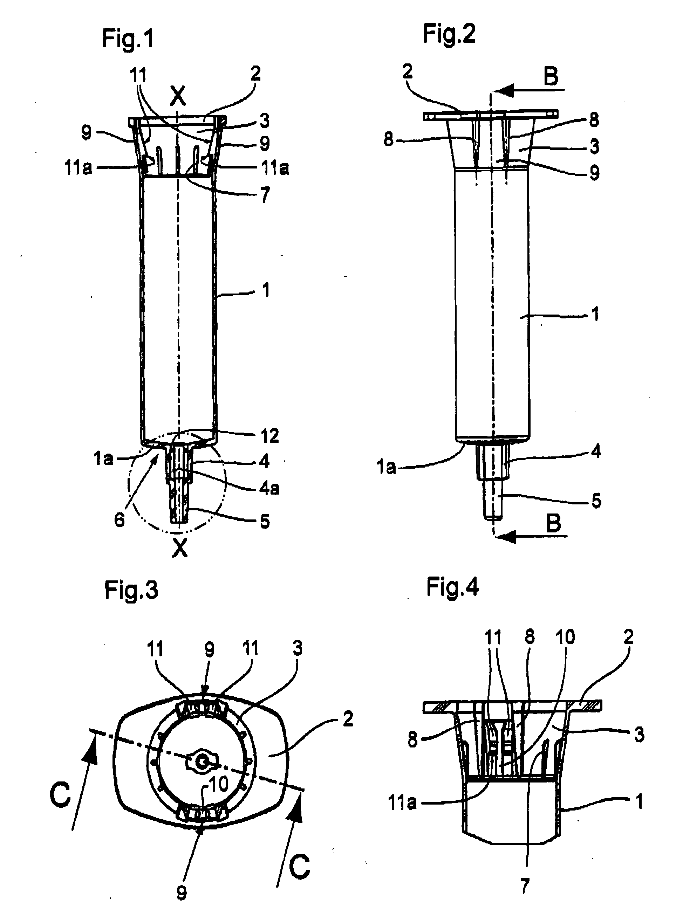

La figure 1 est une vue en coupe longitudinale du corps de la seringue selon la ligne B-B de la figure 2.
La figure 2 est une vue de côté de la seringue.
La figure 3 est une vue en plan de la face arrière ou proximale de la seringue.
La figure 4 est une vue en coupe partielle du corps de la seringue selon la ligne C-C de la figure 3.

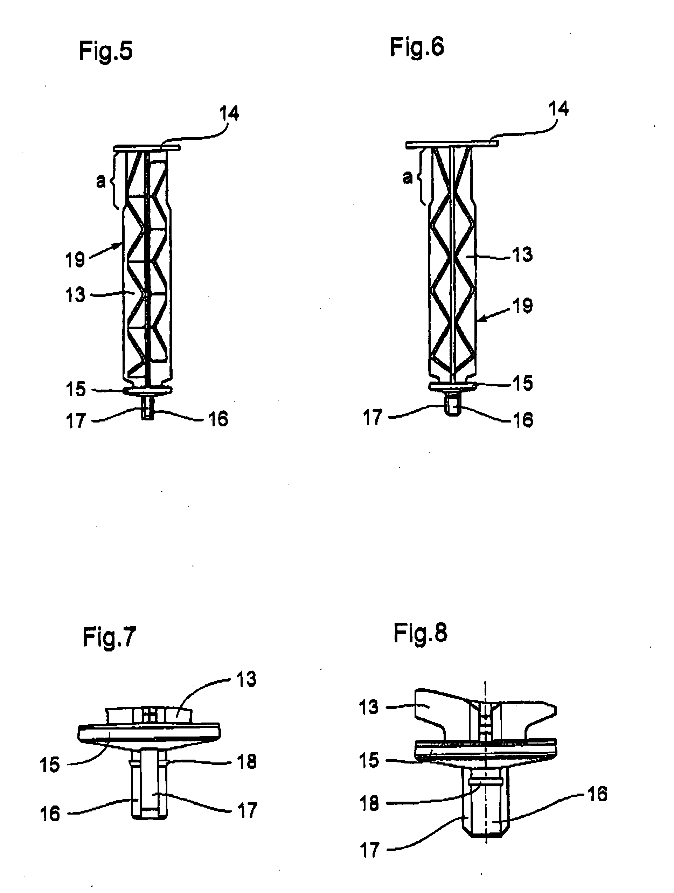

La figure 5 est une vue de côté du piston de la seringue.
La figure 6 est une vue similaire à la figure 5 mais prise à 90°.
La figure 7 est une vue agrandie de la partie distale du piston illustré à la figure 5.
La figure 8 est une vue agrandie de la partie distale du piston illustré à la figure 6.

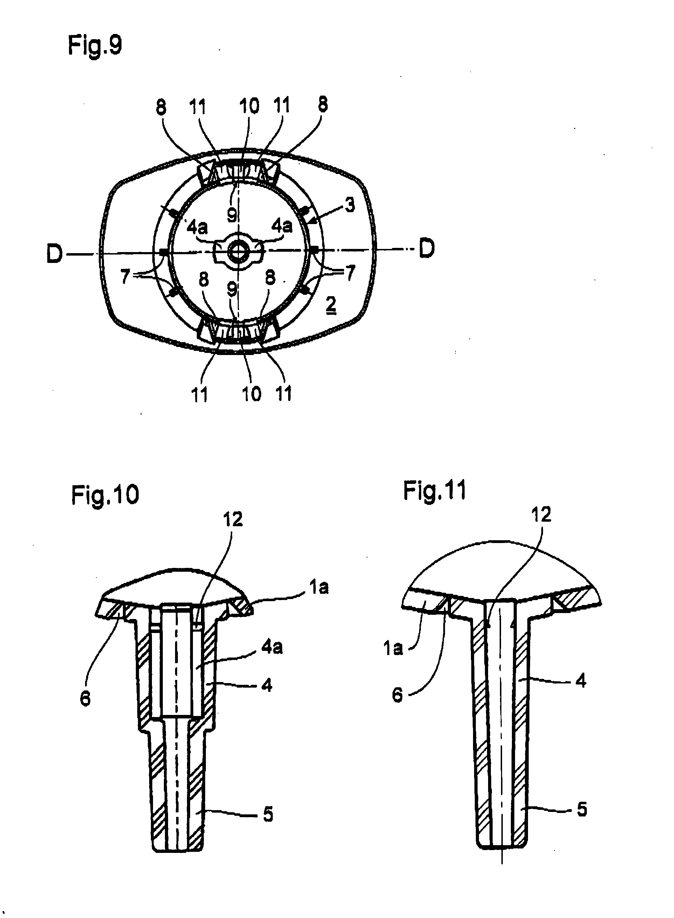

La figure 9 est une vue à plus grande échelle en plan de la partie proximale du corps de seringue.
La figure 11 est une vue à plus grande échelle en coupe axiale de la partie distale du corps de seringue perpendiculaire à la ligne D-D de la figure 9.

La forme d'exécution particulière de la seringue de sécurité illustrée au dessin comporte un corps en forme de cylindre 1 dont l'extrémité arrière est reliée à une plaque 2 par une portion conique 3 allant en s'évasant du cylindre 1 à ladite plaque 2.

L'extrémité distale du cylindre 1 comporte une partie intermédiaire 4, de plus petit diamètre que le cylindre 1, portant la partie terminale 5 destinée à permettre la fixation d'une aiguille sur le corps de la seringue. Cette partie terminale 5, tronconique, est identique à la partie équivalente de seringues usuelles et permet donc de fixer sur le corps de la seringue selon l'invention toutes les aiguilles existantes actuellement commercialisées.

La partie intermédiaire 4 du cylindre 1 comporte des rainures longitudinales 4a débouchant sur sa surface interne. Comme on le verra plus loin, ces rainures longitudinales 4a permettent un accouplement en rotation de cette partie intermédiaire 4 à l'extrémité distale du piston de la seringue en fin d'injection.

La paroi annulaire frontale 1 a du cylindre 1 reliant la partie de plus grand diamètre du cylindre 1 à la partie intermédiaire 4 de ce cylindre comporte une ou plusieurs amorces de rupture 6, par exemple sous la forme d'une gorge annulaire diminuant l'épaisseur de cette paroi annulaire frontale 1 a.

La portion conique 3 du cylindre 1 le reliant à la plaque 2 comporte des nervures de rigidification 7 s'étendant longitudinalement sur une portion de la paroi interne de cette portion conique 3. Cette portion conique 3 est de plus munie d'au moins deux fentes 8 longitudinales délimitant une portion de paroi 9. Dans l'exemple illustré quatre fentes 8 délimitent deux portions de paroi 9 diamétralement opposées. Dans des variantes, trois, quatre ou plus de portions de paroi 9 peuvent être prévues. Ces portions de parois 9, situées entre deux fentes 8, présentent une certaine élasticité et peuvent se déformer élastiquement radialement.

La face interne de chacune des portions de paroi 9 comporte une rainure de guidage 10 longitudinale située dans un plan passant par l'axe X-X du cylindre 1 de la seringue. Cette rainure de guidage 10 est bordée de chaque côté par des nervures 11. L'épaisseur de ces nervures 11 va en augmentant à partir de leur extrémité proximale en direction du cylindre 1, rétrécissant ainsi l'ouverture de la portion conique 3, et se terminant par une chute brutale 11a constituant une butée de retenue arrière dont l'utilité sera décrite plus loin. Les sommets, soit les points les plus épais des nervures 11 où se situe la butée 11a sont situés sur un cercle, concentrique à l'axe X-X du cylindre 1 dont le diamètre est plus petit que le diamètre intérieur du cylindre 1 et du joint 15 du piston.

La paroi interne de la partie intermédiaire 4 du cylindre comporte une première formation d'accouplement axial 12 coopérant, comme on le verra plus loin, avec une seconde formation d'accouplement axial portée par l'extrémité du piston de la seringue permettant d'accoupler, de façon irréversible, en fin d'injection cette partie intermédiaire 4 du cylindre 1 sur l'extrémité du piston de la seringue.

La seringue comporte encore un piston comportant une tige cruciforme 13 munie à son extrémité arrière d'un organe de manoeuvre 14. La partie distale de ce piston comporte un joint 15 venu d'une pièce de fabrication ou de moulage avec la tige 13 et l'organe de manoeuvre 14. Ce joint 15 coulisse sans jeu et de façon étanche dans le cylindre 1. L'extrémité distale de ce piston comporte un organe d'entraînement 16 muni de deux nervures longitudinales 17 destinées à se loger dans les nervures 4a de la partie intermédiaire 4 du cylindre en fin d'injection. Ces nervures longitudinales 17 permettent donc d'accoupler en rotation le piston et la partie intermédiaire 4 du cylindre.

L'organe d'entraînement 16 comporte encore des secondes formations d'accouplement axial 18 coopérant avec les premières formations d'accouplement axial 12 de la partie intermédiaire 4 du cylindre 1 en fin d'injection permettant d'accoupler axialement, de façon non réversible le piston à la partie intermédiaire 4 du cylindre 1.

Le diamètre du joint 15 correspond au diamètre intérieur du cylindre 1 et le piston coulisse ainsi de façon étanche dans le cylindre 1. Le diamètre de ce joint 15 du piston est plus grand que le diamètre d'un cercle, concentrique à l'axe x-x du cylindre 1, passant par les sommets, soit les points de plus forte épaisseur, des nervures de guidage 11 des portions de paroi 9 de la portion conique 3 du cylindre. Ce diamètre du joint 15 du piston est toutefois plus petit que le diamètre de l'ouverture proximale de la portion conique 3 du cylindre 1.

Les arêtes 19 de la tige cruciforme 13 du piston sont, sur la majeure partie de leur longueur, situées sur un cylindre de diamètre égal au diamètre interne du cylindre 1. La partie proximale a de cette tige cruciforme voit ses arêtes 19 se placer sur un cercle de diamètre inférieur.

La seringue ainsi réalisée ne comporte que deux pièces moulées ce qui réduit au maximum son coût de fabrication. Elle ne comporte plus de joint élastomère qui, notamment lors de l'utilisation de cytostatiques à base de platine, peut générer des particules rendant inutilisable l'injection du médicament.

La fabrication et le montage de la seringue selon l'invention s'effectuent de la manière suivante :
a. Le corps de la seringue, soit le cylindre 1, sa partie arrière conique 3, sa partie intermédiaire 4 et sa partie distale 5, est injecté ou moulé en matière plastique. Cette matière plastique est de préférence du polypropylène homopolymère du type PPH 12020 S02 fabriqué par la société Total. Ce matériau offre l'avantage d'être suffisamment ductile pour permettre une bonne déformation et une bonne étanchéité avec le piston et d'être suffisamment cassant pour casser le cylindre dans la zone de rupture 6 lorsque l'utilisateur exerce un effort de rotation entre le piston et le cylindre.
b. Le piston est moulé ou injecté dans une matière plastique qui est de préférence du polyéthylène haute densité du type Bormed HE9621-PH fabriqué par la société Borealis. Ce matériau a l'avantage d'être suffisamment autolubrifiant pour un bon coulissement et une bonne étanchéité avec le cylindre 1 et d'être suffisamment résistant pour résister à l'effort de torsion lorsque l'usager veut provoquer la rupture du cylindre le long de sa zone de rupture 6.
c. Le cylindre et le piston étant réalisés, le piston est introduit dans le cylindre. Ce faisant le joint 15 est introduit dans l'ouverture proximale de la partie conique 3 du cylindre. La périphérie de ce piston vient reposer sur les nervures 11 bordant la ou les rainures de guidage 10 de cette portion conique 3 du cylindre 1. En poussant le piston dans le cylindre, les portions de paroi 9, situées entre une paire de fentes 8, sont déformées vers l'extérieur le piston glissant sur les rampes que forment lesdites nervures 11. Le piston est ajusté angulairement par rapport au cylindre pour que certaines arêtes 19 au moins de la tige 13 du piston soient engagées dans des rainures de guidage 10 du cylindre. Puis le piston est introduit jusqu'au fond du cylindre sans toutefois que ce piston ne soit accouplé à la partie intermédiaire 4 du cylindre 1. Les portions de parois 9 de la partie conique 3 du corps de la seringue reprennent leur position initiale sous l'effet de leur élasticité propre. Ce faisant les butées 11 a sont placées sur un cercle concentrique à l'axe X-X du cylindre 1 mais de diamètre inférieur au diamètre intérieur de ce cylindre 1 et donc du joint 15 du piston.
d. La seringue ainsi moulée et assemblée est emballée et stérilisée.

Ainsi la fabrication et le montage de la seringue sont très faciles et peu onéreux du fait qu'il n'y a que deux pièces à fabriquer et assembler.

Le fonctionnement et l'utilisation de la seringue selon l'invention sont les suivants :
1. La seringue vierge et stérile est sortie de son emballage. Le piston est enfoncé dans le cylindre mais pas suffisamment profondément pour être accouplé à la partie intermédiaire dudit cylindre.
2. L'utilisateur choisit une aiguille correspondant à l'usage qu'il veut en faire et la fixe de façon usuelle sur la partie distale terminale conique 5 du cylindre 1.
3. L'utilisateur déplace le piston vers l'arrière du cylindre pour aspirer le liquide devant être injecté.
4. En tenant la seringue verticale, l'aiguille vers le haut, on évacue l'air contenu dans le cylindre en déplaçant axialement le piston dans le cylindre.
5. L'injection est pratiquée.
6. L'utilisateur enfonce alors le piston complètement dans le cylindre, ce faisant l'organe d'entraînement 16, 17 à l'extrémité distale du piston pénètre dans l'orifice et les rainures 4a de la partie intermédiaire 4 du cylindre 1. L'utilisateur n'a pour ce faire pas à se soucier de l'orientation angulaire du piston par rapport au cylindre car le piston est maintenu et guidé dans la bonne orientation angulaire par les arêtes 19 de sa tige 13 qui coulissent dans les rainures de guidage 10 de la partie conique 3 du cylindre. Arrivé en fin de course le piston est accouplé axialement de façon irréversible à la partie intermédiaire 4 par les formations d'accouplement axial 12 et 18 de cette partie intermédiaire 4 et de l'organe d'entraînement 16 du piston. Ainsi le piston est axialement et angulairement solidaire de la partie intermédiaire 4 du cylindre. Ce faisant en fin de course la partie a de plus faible diamètre de l'arête 19 de la tige 13 du piston est dégagée de la rainure de guidage 10 du cylindre.
7. On déplace angulairement le piston par rapport à la partie proximale de grand diamètre du cylindre 1. Ce faisant l'organe d'entraînement 16 du piston entraîne la partie intermédiaire 4 du cylindre provoquant la rupture de la face annulaire frontale 1 a du cylindre le long de la zone d'affaiblissement 6.
8. L'usager déplace axialement le piston vers l'arrière du cylindre, ce qui provoque la rétraction de la partie intermédiaire 4 séparée du cylindre 1 et de l'aiguille à l'intérieur du cylindre 1 tout en évitant tout contact entre l'usager et l'aiguille utilisée. Lorsque le joint 15 du piston arrive en position arrière il bute contre les butées 11a portées par les nervures 11 de la partie conique 3 du cylindre, de sorte que le piston ne peut pas être extrait du cylindre. La seringue est ainsi sécurisée pour son évacuation et ne peut plus être réutilisée, la partie intermédiaire 4 ayant été séparée du cylindre 1.

Tous les buts proposés ont été atteints car on a réalisé une seringue de sécurité empêchant le personnel de se blesser et interdisant toute réutilisation de la seringue après un premier usage et ceci pour un prix réduit, l'ensemble ne comportant que deux pièces injectées.

De plus, l'utilisation de la seringue est facilitée pour le personnel soignant du fait que l'encliquetage de l'organe d'entraînement 16 du piston dans la partie intermédiaire 4 du cylindre se fait automatiquement puisque l'orientation angulaire de la tige du piston par rapport au cylindre est déterminée par les rainures de guidage 10 du cylindre recevant les arêtes 19 de la tige 13 du piston.

Enfin le piston en fin d'opération ne peut pas être extrait du cylindre 1, le joint 15 du piston venant buter contre les butées 11 a du cylindre.

On notera encore que cette réalisation a permis de supprimer le joint en élastomère du piston.

## Revendications

1. Seringue de sécurité comportant un corps de seringue présentant un cylindre (1) et un piston présentant un joint (15) coulissant à l'intérieur de ce cylindre de façon étanche, dans laquelle le cylindre comporte une partie distale (5) destinée à recevoir une aiguille et une partie intermédiaire (4) dont le diamètre externe est plus petit que le diamètre interne du cylindre (1), la paroi frontale (1a) du cylindre le reliant à la partie intermédiaire (4) comportant une zone de rupture (6), et dans laquelle la partie distale du piston comporte une première formation d'accouplement axial coopérant avec une seconde formation d'accouplement axial que présente la partie intermédiaire (4) du cylindre formant ensemble un accouplement axial irréversible entre le piston et la partie intermédiaire (4) du cylindre (1), cette partie distale du piston comportant en outre une formation d'entraînement en rotation (16, 17) coopérant avec une formation d'entraînement correspondante (4a) que comporte la partie intermédiaire (4) du cylindre (1), le corps de seringue et le piston étant chacun venus d'une seule pièce de fabrication; **caractérisée par le fait que** l'extrémité arrière du cylindre (1) comporte une portion conique (3) allant en s'évasant vers l'arrière et reliant ledit cylindre à une plaque (2); que cette portion conique (3) comporte des fentes longitudinales (8) délimitant au moins une portion de paroi (9) de cette portion conique (3) susceptible de se déplacer élastiquement radialement; et **par le fait que** la face interne de ces portions de paroi (9) comporte d'une part une rainure de guidage longitudinale (10) coopérant avec une arête (19) de la tige (13) du piston et d'autre part au moins une nervure (11) longitudinale présentant une rampe progressant de l'arrière de la portion conique (3) en direction du cylindre et se terminant par une butée (11a) située sur un diamètre plus petit que le diamètre intérieur du cylindre (1) et donc du joint (15) du piston de manière à permettre lors du montage de la seringue l'introduction du piston dans le cylindre par déformation de la portion de paroi (9) mais à interdire tout retrait du piston hors du cylindre.

2. Seringue selon la revendication 1, **caractérisée par le fait que** le corps du cylindre est réalisé en polypropylène homopolymère.

3. Seringue selon la revendication 1, **caractérisée par le fait que** le piston est réalisé en polyéthylène haute densité.

4. Seringue selon l'une des revendications 1 à 3, **caractérisée par le fait que** le piston comporte une tige cruciforme dont des arêtes (19) coopérant avec les rainures (10) de la portion conique (3) du cylindre (1) présentent une plus faible extension dans la partie proximale (a) du piston pour échapper à ladite rainure de guidage (10) lorsque le piston est en fin de course, accouplé avec la partie intermédiaire (4) du cylindre (1).

5. Seringue selon l'une des revendications précédentes, **caractérisée par le fait que** la portion proximale conique (3) du corps de seringue comporte plusieurs, de préférence 2, 3 ou 4 portions de parois (9) déformables élastiquement.

6. Seringue selon la revendication 5, **caractérisée par le fait que** la paroi de la portion proximale conique (3) du corps de la seringue comporte, entre les portions de parois (9) déformables élastiquement, des renforts axiaux de rigidification.

## Patentansprüche

1. Sicherheitsspritze, welche einen Spritzenkörper umfasst, der einen Zylinder (1) und einen eine Dichtung (15) aufweisenden Kolben, der im Inneren dieses Zylinders dicht gleitet, aufweist, wobei der Zylinder einen distalen Teil (5), der dazu bestimmt ist, eine Nadel aufzunehmen, und einen Zwischenteil (4), dessen Außendurchmesser kleiner als der Innendurchmesser des Zylinders (1) ist, aufweist, wobei die vordere Wand (1a) des Zylinders, die diesen mit dem Zwischenteil (4) verbindet, eine Bruchzone (6) aufweist, und wobei der distale Teil des Kolbens eine erste Axialkopplungsformation aufweist, die mit einer zweiten Axialkopplungsformation, welche der Zwischenteil (4) des Zylinders aufweist, zusammenwirkt, wobei beide zusammen eine irreversible axiale Kopplung zwischen dem Kolben und dem Zwischenteil (4) des Zylinders (1) bilden, wobei dieser distale Teil des Kolbens außerdem eine Drehantriebsformation (16, 17) aufweist, die mit einer entsprechenden Antriebsformation (4a), welche der Zwischenteil (4) des Zylinders aufweist, zusammenwirkt, wobei der Spritzenkörper und der Kolben jeweils aus einem einzigen Werkstück hervorgegangen sind; **dadurch gekennzeichnet, dass** das hintere Ende des Zylinders (1) einen konischen Abschnitt (3) aufweist, der sich erweiternd nach hinten verläuft und den Zylinder mit einer Platte (2) verbindet; dass dieser konische Abschnitt (3) Längsschlitze (8) aufweist, die mindestens einen Wandabschnitt (9) dieses konischen Abschnitts (3) begrenzen, der dazu eingerichtet ist, sich elastisch radial zu verschieben; und dadurch, dass die Innenseite dieser Wandabschnitte (9) einerseits eine Längsführungsnut (10) aufweist, die mit einer Kante (19) der Stange (13) des Kolbens zusammenwirkt, und andererseits eine Längsrippe (11), die einen vom hinteren Teil des konischen Abschnitts (3) in Richtung des Zylinders verlaufenden Anstieg aufweist und in einem Anschlag (11a) endet, der sich auf einem Durchmesser befindet, der kleiner als der Innendurchmesser des Zylinders (1) und daher der Dichtung (15) des Kolbens ist, so dass beim Zusammenbau der Spritze die Einführung des Kolbens in den Zylinder durch Verformung des Wandabschnitts (9) ermöglicht wird, jedoch jedes Herausziehen des Kolbens aus dem Zylinder verhindert wird.

2. Spritze nach Anspruch 1, **dadurch gekennzeichnet, dass** der Körper des Zylinders aus homopolymerem Polypropylen hergestellt ist.

3. Spritze nach Anspruch 1, **dadurch gekennzeichnet, dass** der Kolben aus Polyethylen hoher Dichte hergestellt ist.

4. Spritze nach einem der Ansprüche 1 bis 3, **dadurch gekennzeichnet, dass** der Kolben eine kreuzförmige Stange aufweist, deren Kanten (19), die mit den Nuten (10) des konischen Abschnitts (3) des Zylinders (1) zusammenwirken, im proximalen Teil (a) des Kolbens eine geringere Erstreckung aufweisen, um aus der Führungsnut (10) hinauszugleiten, wenn sich der Kolben am Hubende befindet und mit dem Zwischenteil (4) des Zylinders (1) gekoppelt ist.

5. Spritze nach einem der vorhergehenden Ansprüche, **dadurch gekennzeichnet, dass** der konische proximale Abschnitt (3) des Spritzenkörpers mehrere, vorzugsweise 2, 3 oder 4, elastisch verformbare Wandabschnitte (9) aufweist.

6. Spritze nach Anspruch 5, **dadurch gekennzeichnet, dass** die Wand des konischen proximalen Abschnitts (3) des Spritzenkörpers zwischen den elastisch verformbaren Wandabschnitten (9) axiale Versteifungselemente aufweist.

## Claims

1. Safety syringe comprising a syringe body having a cylinder (1) and a piston with a joint (15) sliding inside this cylinder in a tight manner, in which the cylinder comprises a distal portion (5) intended to receive a needle and an intermediate portion (4) the outside diameter of which is smaller than the inside diameter of the cylinder (1), the frontal wall (1a) of the cylinder connecting it to the intermediate portion (4) comprising a breaking zone (6), and in which the distal portion of the piston comprises a first axial coupling formation cooperating with a second axial coupling formation from the intermediate portion (4) of the cylinder forming together a non reversible axial coupling between the piston and the intermediate portion (4) of the cylinder (1), this distal portion of the piston comprising further a rotation driving formation (16,17) cooperating with a corresponding driving formation (4a) of the intermediate portion (4) of the cylinder (1), the body of the syringe and the piston being each made of one piece of manufacture; **characterized by** the fact that the rear end of the cylinder (1) comprises a conical portion (3) widening towards the rear and connecting the said cylinder to a plate (2); that said conical portion (3) comprises longitudinal slots (8) defining at least a wall portion (9) of said conical portion (3) able to displace itself radically elastically; and by the fact that the internal face of these wall portions (9) comprise on the one hand a longitudinal guiding groove (10) cooperating with a ridge (19) of the rod (13) of the piston and on the other hand at least a longitudinal rib (11) having a progressive slape increasing from the rear of the conical portion (3) in direction of the cylinder and terminating by an abutment (11a) located on a diameter smaller than the inside diameter of the cylinder (1) and thus of the joint (15) of the piston in order to permit during the assembly of the syringe the introduction of the piston into the cylinder by a deformation of the wall portion (9) but to hinder any retraction of the piston out of the cylinder.

2. Syringe according to claim 1, **characterized by** the fact that the body of the cylinder is made out of polypropylene homopolymere.

3. Syringe according to claim 1, **characterized by** the fact that the piston is made out of high density polyethylene.

4. Syringe according to one of claims 1 to 3 **characterized by** the fact that the piston comprises a cruciform rod the edges (19) of which cooperate with the grooves (10) of the conical portion (3) of the cylinder (1) having a smaller extension in the proximal portion (a) of the piston to escape to said guiding grooves (10) when the piston reaches the end of its stroke, coupled with the intermediate portion (4) of the cylinder (1).

5. Syringe according to one of the preceeding claims **characterized by** the fact that conical proximal portion (3) of the body of the syringe comprises several, preferably 2, 3 or 4 wall portions (9) which are elastically deformable.

6. Syringe according to claim 5, **characterized by** the fact that the wall of the conical proximal portion (3) of the body of the syringe comprises, between the elastically deformable wall portions (9), axial reinforcements for rigidification.
